Europäisches Patentamt

European Patent Office

Office européen des brevets

(19)

(11) Veröffentlichungsnummer: **0 071 888**
**B1**

(12) **EUROPÄISCHE PATENTSCHRIFT**

(45) Veröffentlichungstag der Patentschrift:
16.07.86

(51) Int. Cl.⁴: **A 61 K 37/14**

(21) Anmeldenummer: 82106849.1

(22) Anmeldetag: 29.07.82

(54) Verfahren zur Gewinnung von hepatitissicheren, sterilen, pyrogenfreien und stromafreien Hämoglobinlösungen.

(30) Priorität: 04.08.81 DE 3130770

(43) Veröffentlichungstag der Anmeldung:
16.02.83 Patentblatt 83/7

(45) Bekanntmachung des Hinweises auf die Patenterteilung:
16.07.86 Patentblatt 86/29

(84) Benannte Vertragsstaaten:
AT BE CH FR GB IT LI NL SE

(56) Entgegenhaltungen:
DE - A - 2 248 475

O. Akerblom und C. F. Högman, Progress in Freeze-Preservation of Red Blood Cells in Sweden (Symosium on Deep-Freeze Preservation of Erythrocytes, Nov. 27-28, 1973 Ffm.)

(73) Patentinhaber: Biotest-Serum-Institut GmbH, Flughafenstrasse 4, D-6000 Frankfurt-Niederrad (DE)

(72) Erfinder: Bonhard, Klaus, Dr. Dipl.-Chem., Georg-Wolff-Strasse 5, D-6450 Hanau 1 (DE)
Erfinder: Eichentopf, Bertram, Dr. Dipl.-Pharm, Im Lauer 7, D-6232 Bad Soden (DE)
Erfinder: Kothe, Norbert, Dr. Dipl.-Chem., Friedrich-Ebert-Strasse 21, D-6242 Kronberg (DE)

(74) Vertreter: Beil, Walter, Dr. et al, BEIL, WOLFF & BEIL Rechtsanwälte Adelonstrasse 58, D-6230 Frankfurt am Main 80 (DE)

## Beschreibung

Die Erfindung betrifft ein Verfahren zur Gewinnung von hepatitissicheren, sterilen, pyrogen- und stromafreien Hämoglobinlösungen mit niedrigem Kaliumgehalt, bei dem man wenige bis 25 Minuten mit einer verdünnten β-Propiolacton-Lösung behandelt, das durch Einrühren der Erythrozyten in destilliertes Wasser erhaltene Hämolysat dann mit einem Kationenaustauscher in H⁺-Form behandelt, bis der pH-Wert auf 5,0 bis 5,5 abgesunken ist, vom Harz und der ausgefällten Stromamasse durch Zentrifugieren und/oder Filtrieren befreit, den pH-Wert auf 7,2 bis 7,6 sowie die gewünschte Hämoglobinkonzentration einstellt, gegebenenfalls die je nach Zweck der Anwendung erforderlichen Zusätze zugibt und sterilfiltriert.

Hämoglobinlösungen sind in der Lage, unabhängig vom Erythrozyten in vivo Sauerstoff zu transportieren. Für die Verträglichkeit solcher Lösungen ist es jedoch nötig, Plasma und Zellbestandteile des Blutes, aus dem das Hämoglobin durch Hämolyse freigesetzt wird, möglichst vollständig zu entfernen (Plasmaproteine, Stroma, Leukozyten, Thrombozyten).

Aus der DE-PS 2 248 475 ist ein Verfahren zur Herstellung derartiger Hämoglobinlösungen bekannt, bei dem die Trennung von Plasmaproteinen und Erythrozyten durch Aufschwemmen der Erythrozyten in physiologischen Elektrolytlösungen und nachfolgender Separation der Erythrozyten durch Zentrifugieren in diskontinuierlich arbeitenden Zentrifugen erfolgt.

In Frank De Venuto, Harold J. Friedmann, J. Ryan Neville, Carl C. Peck «Appraisal of Hemoglobin Solution as a Blood Substitute», Surgery, Gynecology & Obstetrics, Sept. 1979 Vol. 149, S. 417–436, sind unterschiedliche Verfahren beschrieben, nach denen das Hämoglobin durch Auskristallisieren gewonnen wird.

Sowohl die zur Waschung der Erythrozyten notwendige diskontinuierliche Zentrifugation (Blutzentrifuge), als auch die bei Kristallisationsverfahren notwendige Dialyse sind ungeeignete Verfahrensschritte zur Herstellung grösserer Mengen Hämoglobinlösung, wie sie zur klinischen Verwendung benötigt würden. Übliche diskontinuierlich arbeitende Zentrifugen haben beispielsweise ein Fassungsvermögen von nur ca. 6 Litern. Der Einsatz einer Vielzahl von diskontinuierlich arbeitenden Zentrifugen wäre äusserst unwirtschaftlich.

Die Erythrozytenwaschungen mit Elektrolytlösungen bedingen einen hohen Elektrolytgehalt des Endproduktes, was für spezielle Verwendungszwecke, z.B. Perfusion der Herzkranzgefässe bei Operationen am offenen Herzen (Kardioplegie), von Nachteil wäre.

Bis auf das in der DE-PS 2 248 475 beschriebene Verfahren ist bei allen übrigen Herstellungsmethoden die Übertragung einer Serumhepatitis bei Anwendung nicht auszuschliessen.

Jedoch auch die in der DE-PS 2 248 475 beschriebene Sterilisationsmethode mit β-Propiolacton (β-PL) weist den Nachteil auf, dass diese Behandlung als erster Prozessschritt erfolgt, was bedeutet, dass die eingesetzte β-PL-Menge auf je nach Herstellung des Erythrozytenkonzentrats verbleibende Restmengen von Plasmaproteinen einwirkt. Dadurch wirkt auf die Erythrozyten, je nach Restplasmamenge, eine unterschiedliche β-PL-Konzentration ein.

Es sind zwar auch kontinuierlich arbeitende Zentrifugen zur Trennung von grossen Mengen Blutes in Zellbestandteile und Plasma bekannt und werden zum Beispiel in Schlachthöfen zur Gewinnung eines hämoglobinfreien Plasmas eingesetzt. Bei diesem Verfahren werden jedoch die Erythrozyten so stark geschädigt, dass sie speziell bei mehrmaligem Durchlauf nahezu vollständig zerstört werden. Deshalb ist diese kontinuierliche Zentrifugation zum Waschen von Erythrozyten nicht geeignet.

Der Erfindung liegt die Aufgabe zugrunde, ein Verfahren zur Gewinnung von hepatitissicheren, sterilen, pyrogen- und stromafreien Hämoglobinlösungen bereitzustellen, mit Hilfe dessen derartige Hämoglobinlösungen für unterschiedliche Anwendungsgebiete auf wirtschaftliche Weise in so grossen Mengen hergestellt werden können, wie sie für die klinische Anwendung benötigt werden und das die schonende Waschung von Erythrozyten gestattet.

Diese Aufgabe wird erfindungsgemäss dadurch gelöst, dass man vor der β-Propiolactonbehandlung ein Erythrozytenkonzentrat mit dem 1 bis 6-fachen Volumen einer 5 bis 15%igen wässrigen Lösung eines Zuckers, Zuckeralkohols oder hochmolekularen Kolloids unter Rühren versetzt, den pH-Wert der Suspension mit einer Säure, die als Salz physiologisch verträglich ist auf 5 bis 6,5 einstellt, nach Abschalten des Rührers die Erythrozyten sedimentieren lässt, den Überstand entfernt, gegebenenfalls vorstehenden Waschprozess bei dem erhaltenen Erythrozytensediment ein oder mehrere Male wiederholt, den pH-Wert des Sediments auf 7 bis 8 einstellt, das Sediment homogenisiert und dessen Hämatokrit auf 55 bis 60% einstellt und nach der β-Propiolactonbehandlung den vorangegangenen Waschprozess ein oder mehrere Male wiederholt.

Erythrozyten in einer Suspension haben das Bestreben zu sedimentieren. Dieses Phänomen wird als Blutsenkung bezeichnet und in der medizinischen Diagnostik zur Erkennung entzündlicher Prozesse verwendet. Die normale Blutsenkung beträgt ca. 5 mm/Stunde. Durch Zusatz verschiedener Substanzen lässt sich die Erythrozytensedimentation beschleunigen. Aus der Literatur sind Anwendungsbeispiele für Sedimentationsbeschleuniger bekannt. So werden zum Beispiel gemäss R.G. Strauss «In vitro Comparison of the Erythrocyte Sedimenting Properties of Dextran, Hydroxyethyl Starch and a New Low-Molecular-Weight Hydroxyethyl Starch», Vox Sang. 37:268–271 (1979)(3) zur Herstellung von Thrombozyten- und Leukozytenkonzentraten hochmolekulare Kolloide verwendet. Gemäss O. Akerblom and C.F. Högman «Progress in Freeze-Preservation of Red Blood Cells in Sweden» (Symposium on Deep-Freeze Preservation of Erythrocytes, November 27–28, 1973, Frankfurt/Main)(4) finden Zucker oder Zuckeralkohole Verwendung bei der Entfernung von Glycerin aus Erythrozytenkonzentraten, die nach Tiefgefrierlagerung aufgetaut wurden. Zum Einsatz kommen hierbei Mischungen von Glukose und Fruktose in unterschiedlichen Konzentrationen, wobei bestimmte Mischungsverhältnisse vorgegeben sind. Die Elektrolytkonzentration ist auf 10 bis max. 15 mmol/l begrenzt. Aus (4) ist somit zwar die Verwendung von Zucker oder Zuckeralkoholen als Sedimentationsbeschleuniger bekannt, jedoch wurde die Sedimentationsmethode unter Verwendung dieser Beschleuniger bisher bei der Herstellung von Hämoglobinlösungen (20 Jahre) niemals probiert. Diese Druckschrift gibt auch keinen Hinweis darauf, dass bei der Sedimentierung in Gegenwart von Zuckern oder Zuckeralkoholen ein bestimmter pH-Bereich eingestellt werden muss.

Bei dem erfindungsgemässen Verfahren zeigte sich im Gegensatz zu den erwähnten Literaturangaben, dass die Sedimentation mit nur einem Zucker bzw. Zuckeralkohol möglich ist. Eine bestimmte Mischung mehrerer Zucker ist bei dem erfindungsgemässen Verfahren nicht notwendig und im Hinblick auf die breite Einsatzmöglichkeit, wie sie vorstehend bereits dargelegt wurde, nicht wünschenswert.

Desgleichen genügt es, mehrfache Sedimentationen mit nur einer einzigen Zuckerkonzentration durchzuführen, wenn bei entsprechend niedrigen pH-Werten gearbeitet wird.

Erfindungsgemäss werden Erythrozytenkonzentrate, die wegen Überschreitung der maximalen Lagerzeit nicht mehr zur Transfusion geeignet sind, blutgruppenunabhängig gepoolt und mit einem 1–6-fachen, vorzugsweise 2,5-fachen Volumen einer 5–15%igen, vorzugsweise 10%igen, Zucker- bzw. Zuckeralkohollösung oder eines hochmolekularen Kolloids unter Rühren versetzt. Als Zucker bzw. Zuckeralkohole können Glukose, Mannit, Fruktose, Sorbit, Xylit sowie Disaccharide wie Saccharose und Maltose verwendet werden. Als hochmolekulare Kolloide eignen sich Hydroxyäthylstärke oder Dextran. Der pH-Wert der Suspension wird mit einer physiologisch verträglichen Säure, z.B. 1 n Salzsäure auf 5–6,5, vorzugsweise 5,4, eingestellt. Der pH-Wert von 5,4 hat sich besonders bei höherem Elektrolytgehalt im Ausgangsmaterial als günstig erwiesen. Die Temperatur der Suspension wird vorzugsweise während des gesamten Prozesses bei 2–18 °C, besonders bevorzugt 10 °C, gehalten. Nach Abschalten des Rührers lässt man die Erythrozyten vorzugsweise 15 Minuten bis 10 Stunden, besonders bevorzugt 60 Minuten, sedimentieren. Nach der Sedimentationszeit entfernt man den Überstand, bestimmt das Volumen des Sediments und wiederholt den Waschprozess. Das so erhaltene Erythrozytensediment wird durch Einstellen des pH-Wertes auf 7–8, vorzugsweise 7,4 desaggregiert und durch Rühren homogenisiert. Der Hämotokrit des Sediments wird mit der Sedimentationslösung auf 55–60% eingestellt. Dann wird eine β-Propiolac-

tonlösung rasch unter Rühren zugesetzt. Vorzugsweise verwendet man pro Liter Sediment 0,166 l 1,5%ige mit der Sedimentationslösung frisch hergestellte β-Propiolactonlösung. Die zwei- oder mehrmalige Waschung der Erythrozyten vor der Zugabe von β-Propiolacton hat gegenüber früher beschriebenen Verfahren den Vorteil, dass keine nennenswerten Mengen an Restplasma vorhanden sind, die die Sterilisationswirkung des β-Propiolactons beeinflussen. Dies bedeutet, dass eine immer gleichbleibende β-Propiolactonkonzentration auf ein bestimmtes Erythrozytenvolumen einwirkt.

Nach einer Einwirkungszeit von 20 Minuten hat das β-Propiolacton nahezu vollständig reagiert. Zur Entfernung der Reaktionsprodukte werden noch eine oder mehrere, vorzugsweise drei Waschungen wie oben beschrieben durchgeführt. Die gewaschenen Erythrozyten werden durch Zugabe von destilliertem Wasser hämolysiert. Die Hämolyse erfolgt vorzugsweise durch Zugabe des 1,5- bis 5-fachen, besonders bevorzugt 2,5-fachen Volumens destillierten Wassers bei pH 7. Zur Abtrennung des Stromas wird das Hämolysat mit einem Kationenaustauscherharz in $H^+$-Form, zweckmässigerweise in Mengen von 50–150, vorzugsweise 80 mval/l, behandelt, bis der pH-Wert auf 5,0 bis 5,5, vorzugsweise 5,1 abgesunken ist. Besonders geeignete Kationenaustauscherharze sind saure Polystyrolsulfonat – Kationenaustauscher, die eine Austauscherkapazität von 4–5 mval je g trockenes Harz haben und andere Polystyrolharze, die sich im wesentlichen hinsichtlich Hitzestabilität, Körnung, Pigmentgehalt und Austauschkapazität voneinander unterscheiden. Näheres in K. Dorfner: «Ionenaustauscher», Walter de Gruyter, Berlin (1964).

Nach Anhebung des pH-Wertes auf 5,2 bis 6, vorzugsweise 5,65, wird der Ionenaustauscher abfiltriert und das ausgefällte Stroma durch kontinuierliche Zentrifugation und/oder Filtrieren entfernt. Nach Einstellen des pH-Wertes auf 7,2 bis 7,6, vorzugsweise 7,4 und Einstellung der gewünschten Hämoglobinkonzentration, sowie gegebenenfalls der Zugabe weiterer Zusätze, wird die Lösung vor der Sterilfiltration nochmals zentrifugiert und/oder vorfiltriert.

Zusätze können je nach Verwendungszweck Elektrolyte bzw. Effektoren zur Verbesserung der Sauerstoffabgabe des Hämoglobins, wie z.B. 2,3-Diphosphoglycerat, Pyrridoxalphosphat oder Inosithexaphosphat sein.

Mit dem erfindungsgemässen Verfahren lassen sich je nach Grösse des Sedimentationsgefässes und der zur Verfügung stehenden Zentrifugenkapazität, beliebige Mengen in einem Ansatz herstellen.

Das erfindungsgemässe Produkt lässt sich je nach Art der Zusätze zur Perfusion der Herzkranzgefässe (Cardioplegie) oder abgetrennter bzw. abgequetschter Extremitäten verwenden oder aber als Ausgangsprodukt zur Herstellung chemisch modifizierter Hämoglobinlösungen für Infusionszwecke, wie beispielsweise in DE-PS 2 449 885 oder 2 617 822 beschrieben.

So erfordert beispielsweise eine cardioplegische Lösung einen geringen Na- und Glukosegehalt, während zur Extremitätenperfusion physiologische Na-Werte und ein hoher Glukosegehalt erwünscht sind.

Nach heutigem Stand der Wissenschaft müssen für die Anwendung am Menschen Human-Erythrozyten verwendet werden.

Beispiel 1:

111 Erythrozytenkonzentrate wurden in einem kühlbaren 160 l VA-Gefäss mit Rührwerk gepoolt. Es ergab sich ein Volumen von 26 Litern. Die Temperatur wurde während des gesamten Verfahrens bei ca. 10 °C gehalten, nach Zusatz von 65 l 10%iger Glukoselösung und Einstellen des pH-Wertes auf 5,4 wurde 10 Minuten gerührt. Anschliessend liess man 45 min sedimentieren und saugte den Überstand ab. Das Sediment enthielt 18,4% Hämoglobin und der Hämatokrit betrug 49%. Zur zweiten Waschung füllte man mit 112 l 10%iger Glukoselösung auf, korrigierte den pH-Wert auf 5,4, rührte 10 Minuten und liess erneut 45 Minuten sedimentieren.

Das Sediment (28 l, Hämatokrit 67%, Hb 25,5%) wurde nach Anheben des pH-Wertes auf 7,4 mit 1 N NaOH unter Rühren homogenisiert. Der Hämatokrit wurde mit 10% Glukoselösung auf 55–60% (59%) eingestellt. Nun fügte man 4,9 l 1,5%ige β-Propiolactonlösung in 10%iger Glukose rasch hinzu und liess unter Rühren 20 Minuten einwirken, wobei der pH-Wert auf 7,14 absank. Zur dritten Sedimentation wurde mit 10%iger Glukoselösung auf 100 Liter aufgefüllt und der pH-Wert erneut auf 5,4 eingestellt. Die Sedimentation erfolgte ebenso wie die 4. und 5. Sedimentation in der oben beschriebenen Weise. Die Volumina des dritten, vierten und fünften Sediments betrugen 31,27 und 24 Liter. Der pH-Wert des fünften Sediments wurde mit 1 N NaOH auf 7,0 eingestellt. Die Hämolyse erfolgte durch Vermischen mit 60 Litern destilliertem Wasser. Nach 30 Minuten intensivem Rühren wurde die Vollständigkeit der Hämolyse überprüft. Zur Entfernung des Stromas wurden zunächst 143 g NaCl, sodann 2,94 kg hochreines (pA-Qualität) Kationenaustauscherharz mit einer Kapazität von 4 mval pro g Trockensubstanz, einem Divinylbenzolanteil von 8% bei der Herstellung seiner Polystyrolmatrix und einer Körnung zwischen 297 und 840 µm in H$^+$-Form zugegeben. Der pH-Wert sank kontinuierlich ab.

Bei Erreichen von pH 5,1 wurden 2,5 Liter NaOH 1 n langsam unter Rühren zugegeben. Der pH-Wert stieg auf 5,65. Der Ionenaustauscher wurde über ein grobmaschiges Sieb, das ausgefallene Stroma durch kontinuierliche Zentrifugation bei ca. 5000 g abgetrennt. Der pH-Wert der so geklärten Lösung wurde durch Zugabe von 1,4 Litern 1 n NaOH auf 7,4 eingestellt. Man fügte 247 g NaCl, 110 g NaHCO$_3$ und 715 g Glukose zu und zentrifugierte erneut.

Die abschliessende Sterilfiltration erfolgte in zweifacher Weise, nämlich zunächst über Tiefenfilterschichten, sodann über Membranfilter, wie sie üblicherweise für Sterilfiltration verwendet werden.

Ausbeute: 60 Liter
Hämoglobin: 8%

Beispiel 2:

122 Erythrozytenkonzentrate wurden in gleicher Weise wie in Beispiel 1 gepoolt. Das Volumen betrug 29 Liter, die Temperatur 10 °C. Nach Zusatz von 72,5 Litern 10%iger Mannitlösung und Einstellen des pH-Wertes auf 5,4 mit 1 N HCl wurde 10 Minuten gerührt. Die Sedimentationszeit betrug 45 Minuten, das Sediment enthielt 22,8% Hämoglobin und wies einen Hämatokrit von 45% auf. Die 2. Waschung erfolgte mit 82,5 Litern 10%iger Mannitlösung. Das Volumen des Sediments betrug 24 Liter. Die β-Propiolactonbehandlung erfolgte in gleicher Weise wie in Beispiel 1. Die Mengen an 10%iger Mannitlösung für die dritte, vierte und fünfte Waschung betrugen 75, 65 und 63 Liter, die Sedimentvolumina 26, 25 und 17 Liter. Nun verfuhr man weiter wie in Beispiel 1.

Ausbeute: 35 Liter
Hb-Gehalt: 9%

Beispiel 3:
1. Waschlösung:
6% Hydroxyäthylstärke
in 0,9% NaCl
2. Waschlösung:
6% Hydroxyäthylstärke
in 1,3% NaHCO$_3$

1 Liter Waschlösung 1 wurde in einem 10 l Gefäss vorgelegt und 7 Erythrozytenkonzentrate (insgesamt ca. 1,7 l) unter Rühren hinzugefügt. Anschliessend gab man weitere 2,5 l Waschlösung 1 zur Erythrozytensuspension hinzu, so dass das Verhältnis von Erythrozytenkonzentrat zur Waschlösung 1 : 2 betrug. Innerhalb von 75 Minuten sedimentierten die Zellen, woraus ein Verhältnis von Überstand zum Sediment von ca. 1 : 1 resultierte. Der Überstand wurde abgesaugt, das Sediment mit 3,5 l Waschlösung 2 unter Rühren resuspendiert und anschliessend 60 Minuten der Sedimentation überlassen. Der Überstand, der noch 0,18% Protein enthielt, wurde entfernt. Zum Sediment, das einen Hämatokrit von 57,5% aufwies, fügte man 415 ml Waschlösung 1, die 1,5%ig an β-Propiolacton war, zu und liess 20 Minuten unter Rühren einwirken. Anschliessend wurde mit Waschlösung 2 auf 6 l aufgefüllt.

Nach 60 Minuten saugte man den Überstand, der 0,15% Hämoglobin enthielt, ab und wiederholte die Sedimentation mit Waschlösung 1. Man verfuhr anschliessend wie in Beispiel 1.

Ausbeute: 5,5 l
Hämoglobin: 7%

**Patentansprüche**

1. Verfahren zur Gewinnung von hepatitissicheren, sterilen, pyrogen- und stromafreien Hämoglobinlösungen mit niedrigem Kaliumgehalt, bei dem man wenige bis 25 Minuten mit einer verdünnten β-Propiolacton-Lösung behandelt, das durch Einrühren der Erythrozyten in destilliertes Wasser erhaltene Hämolysat dann mit einem Kationenaustauscher in H$^+$-Form behandelt, bis der pH-Wert auf 5,0 bis 5,5 abgesunken ist, vom Harz und der ausgefällten Stromamasse durch Zentrifugieren und/oder Filtrieren befreit, den pH-Wert auf 7,2 bis 7,6 sowie die gewünschte Hämoglobinkonzentration einstellt, gegebenenfalls die je nach Zweck der Anwendung erforderlichen Zusätze zugibt und sterilfiltriert, dadurch gekennzeichnet, dass man vor der β-Propiolactonbehandlung ein Erythrozytenkonzentrat mit dem 1 bis 6-fachen Volumen einer 5 bis 15%igen wässrigen Lösung eines Zuckers, Zuckeralkohols oder hochmolekularen Kolloids unter Rühren versetzt, den pH-Wert der Suspension mit einer Säure, die als Salz physiologisch verträglich ist auf 5 bis 6,5 einstellt, nach Abschalten des Rührers die Erythrozyten sedimentieren lässt, den Überstand entfernt, gegebenenfalls Waschprozess bei dem erhaltenen Erythrozytensediment ein oder mehrere Male wiederholt, den pH-Wert des Sediments auf 7 bis 8 einstellt, das Sediment homogenisiert und dessen Hämatokrit auf 55 bis 60% einstellt und nach der β-Propiolactonbehandlung den vorangegangenen Waschprozess ein oder mehrere Male wiederholt.

2. Verfahren nach Anspruch 1, dadurch gekennzeichnet, dass man die Temperatur der Suspension während der Waschprozesse auf 2 bis 18°C hält.

3. Verfahren nach einem der vorstehenden Ansprüche, dadurch gekennzeichnet, dass man den pH-Wert der Suspension vor dem Sedimentieren auf 5,4 einstellt.

4. Verfahren nach einem der vorstehenden Ansprüche, dadurch gekennzeichnet, dass man die Erythrozyten 15 Minuten bis 10 Stunden sedimentieren lässt.

5. Verfahren nach einem der vorstehenden Ansprüche, dadurch gekennzeichnet, dass man den pH-Wert des Sediments vor der β-Propiolactonbehandlung auf 7,4 einstellt.

6. Verfahren nach einem der vorstehenden Ansprüche, dadurch gekennzeichnet, dass man zwei Erythrozytenwaschungen vor und drei nach der β-Propiolactonbehandlung durchführt.

## Claims

1. Process for obtaining hepatitis-safe, sterile, pyrogen- and stroma-free hemoglobin solutions of low potassium content which comprises treating for several to 25 minutes with a dilute solution of β-propiolactone, thereupon treating the hemolyzate obtained by stirring the erythrocytes into distilled water with a cation exchanger in H$^+$ form until the pH has fallen to 5.0 to 5.5, freeing from the resin and from the precipitated mass of stroma by centrifugation and/or filtration, adjusting the pH to 7.2 to 7.6 and adjusting the desired hemoglobin concentration, adding the additives required for the intended application if desired, and sterilising by filtration, characterized in that prior to the β-propiolactone treatment 1 to 6 times its volume of an aqueous 5 to 15% solution of a sugar, sugar alcohol or hig-molecular-weight colloid is added to an erythrocyte concentrate during stirring, the pH of the suspension is adjusted to 5 to 6.5 with an acid which is physiologically acceptable in salt form, the erythrocytes are allowed to settle after shuting off the stirrer, the supernatant is removed, the above washing process is repeated one or more times on the erythrocyte sediment if desired, the pH of the sediment is adjusted to 7 to 8, the sediment is homogenized and its hematocrit is adjusted to 55 to 60% and after the β-propiolactone treatment the above washing process is repeated one or more times.

2. Process according to claim 1, characterized in that the temperature of the suspension is maintained from 2 to 18°C during the washing process.

3. Process according to one of the preceding claims, characterized in that the pH of the suspension is adjusted to 5.4 prior to the settling.

4. Process according to one of the preceding claims, characterized in that the erythrocytes are allowed to settle for from 15 minutes to 10 hours.

5. Process according to one of the preceding claims, characterized in that the pH of the sediment is adjusted to 7.4 prior to the β-propiolactone treatment.

6. Process according to one of the preceding claims, characterized in that two washings of the erythrocytes are performed before and three after the β-propiolactone treatment.

## Revendications

1. Procédé de préparation de solutions stériles d'hémoglobine exemptes d'hépatite, de pyrétogène, et de stroma, à faible teneur en potassium, selon lequel on traite de petites quantités jusqu'à 25 minutes par une solution diluée de béta-propiolactone qui traite l'hémolysat obtenu par mise en contact des globules rouges avec de l'eau distillée puis avec un échangeur de cations sous forme −H$^+$ jusqu'à abaissement du pH entre 5 et 5,5, l'hémolysat est alors débarrassé de la résine et de la masse de stroma par centrifugation ou filtration, le pH est amené entre 7,2 et 7,6 et la concentration en hémoglobine est amenée aussi à la valeur désirée, éventuellement on ajoute les additifs nécessités par chaque utilisation et on filtre de façon stérile, procédé caractérisé en ce qu'avant le traitement par la béta-propiolactone, un concentrat de globules rouges est additionné à raison de 1 à 6 volumes d'une solution aqueuse à 5 à 15% de sucre, d'alcool de sucre ou de colloïde à haut poids moléculaire, sous agitation, on ajuste le pH de la suspension par un acide qui comme sel est compatible physiologiquement, entre 5 et 5,6, on laisse sédimenter les globules rouges après cessation de l'agitation, on élimine la partie supérieure, on renouvelle éventuellement le processus de lavage précédent une ou plusieurs fois sur les globules rouges obtenus, on amène le pH du sédiment entre 7 et 8, on homogénéise le sédiment et on ajuste son hématocrite à une valeur comprise entre 55 et 60%, et après le traitement par la béta-propiolactone on renouvelle une ou plusieurs fois le processus de lavage ci-dessus.

2. Procédé selon la revendication 1, caractérisé en ce qu'on maintient la température entre 2 et 18 °C pendant le processus de lavage.

3. Procédé selon l'une des revendications précédentes, caractérisé en ce qu'on ajuste le pH de la suspension de sédiment à 5,4.

4. Procédé selon l'une des revendications précédentes, caractérisé en ce qu'on laisse sédimenter les globules rouges de 15 minutes à 10 heures.

5. Procédé selon l'une des revendications précédentes, caractérisé en ce que le pH du sédiment est ajusté à 7,4 avant le traitement par la béta-propiolactone.

6. Procédé selon l'une des revendications précédentes, caractérisé en ce que l'on effectue deux lavages des globules rouges avant le traitement par la béta-propiolactone et trois lavages après ce dernier.